# EUROPEAN PATENT APPLICATION

(11) **EP 4 289 943 A1**
(43) Date of publication of application: **13.12.2023**
(21) Application number: 22749798.9
(22) Date of filing: 03.02.2022
(51) Int. Cl.: C12N 5/10, C12N 1/15, C12N 1/19, C12N 1/21, C12N 15/55, C12N 15/63, C12P 21/02

(54) **PROTEIN DEAMIDATING ENZYME**

(30) Priority: 03.02.2021 JP 2021016088
(71) Applicant: Amano Enzyme Inc., Nagoya-shi Aichi 460-8630 (JP)
(72) Inventor: MATSUBARA, Hirotaka, Kakamigahara-shi, Gifu 509-0109 (JP); HIURA, Keita, Kakamigahara-shi, Gifu 509-0109 (JP)
(74) Representative: Müller-Boré & Partner Patentanwälte PartG mbB
(86) International application number: PCT/JP2022/004322
(87) International publication number: WO 2022/168927

(57) **Abstract**

This invention provides a novel protein deamidating enzyme. This protein deamidating enzyme includes a polypeptide containing an amino acid sequence shown in any of sequence numbers 1-11, or a subsequence thereof (the amino acid sequence of positions 338-515 in sequence number 1, positions 381-564 in sequence number 2, positions 122-309 in sequence number 4, positions 1-146 in sequence number 5, positions 19-235 in sequence number 6, positions 19-205 in sequence number 6, positions 10-303 in sequence number 7, positions 10-186 in sequence number 7, positions 162-426 in sequence number 9, or positions 162-341 in sequence number 9), a polypeptide which contains an amino acid sequence obtained by substituting, adding, inserting, or deleting one or multiple amino acids in the amino acid sequence in any of sequence numbers 1-11 or a subsequence thereof, and which has protein glutaminase activity, or a polypeptide which contains an amino acid sequence that has a 70% or higher sequence identity with an amino acid sequence shown in any of sequence number 1-11, or a subsequence thereof, and that has protein glutaminase activity.

## Description

### TECHNICAL FIELD

The present invention relates to a novel protein deamidating enzyme.

### BACKGROUND ART

A protein deamidating enzyme is an enzyme that acts on a protein, which is a macromolecule, and catalyzes a reaction of decomposing (that is, deamidating) an amide group-containing side chain without cleaving peptide bonds or crosslinking the protein. The protein deamidating enzyme deamidates a glutamine residue in a protein to generate a negatively charged carboxyl group, thus causing various changes in the characteristics of the protein. For example, an increase in hydration force and an increase in electrostatic repulsive force due to a decrease in the isoelectric point of the protein reduce the interaction between protein molecules (that is, reduce the associative property) and thus increase the solubility and water dispersibility of the protein. In addition, the exposure of an internal hydrophobic region due to the change in the higher order structure of the protein imparts surface activity to the protein and thus improves the emulsifying power, emulsion stability, foaming property, and foam stability of the protein. The protein deamidating enzyme can greatly change the characteristics of the protein in this way and has therefore dramatically increased the range of use of proteins. For this reason, protein deamidating enzymes are very useful and have attracted high interest in the art.

The first discovered protein deamidating enzyme is a protein-glutaminase found from Chryseobacterium proteolyticum in 2000 (Non-Patent Document 1). However, protein-glutaminases are also extremely peculiar enzymes that, despite their high utility and interest, have not been newly discovered over a long time of the ensuing 20 years. A protein-glutaminase was found from Bacteroides helcogenes after about 20 years (Non-Patent Document 2), but as of January 2021, a protein-glutaminase industrially used is only the protein-glutaminase derived from C. proteolyticum.

### PRIOR ART DOCUMENTS

### NON-PATENT DOCUMENTS

Non-Patent Document 1: A novel protein-deamidating enzyme from Chryseobacterium proteolyticum sp. nov., a newly isolated bacterium from soil. Applied and Environmental Microbiology 2000; 66(8): 3337-43
Non-Patent Document 2: A novel protein glutaminase from Bacteroides helcogenes-characterization and comparison, Applied Microbiology and Biotechnology (2020) 104:187-199

### SUMMARY OF THE INVENTION

### PROBLEMS TO BE SOLVED BY THE INVENTION

Since protein deamidating enzymes having protein-glutaminase activity have not been discovered for many years after the report of the protein-glutaminase derived from C. proteolyticum, it has been established as general technical common knowledge of those skilled in the art that those protein deamidating enzymes are peculiar enzymes that hardly exist in nature and cannot be obtained by ordinary screening. An example in which a protein deamidating enzyme having protein-glutaminase activity other than the protein-glutaminase derived from C. proteolyticum was found is only one example of the protein-glutaminase derived from B. helcogenes, and the perception of those skilled in the art on these enzymes still remains restricted to the technical common knowledge described above.

Under such circumstances, the present invention dares to aim to find a novel protein deamidating enzyme.

### MEANS FOR SOLVING THE PROBLEM

The present inventors have intensively studied to find a novel protein deamidating enzyme, but no enzyme having protein deamidating activity has been found from candidates having high sequence identity with known protein deamidating enzymes. However, it has been found by chance that an enzyme having protein deamidating activity is present in a protein group having low sequence identity with known protein deamidating enzymes. The present invention has been completed on the basis of these findings.

That is, the present invention provides the invention of the following aspects.

Item 1. A protein deamidating enzyme including a polypeptide as defined in any of (a) to (c) below:
(a) a polypeptide including the amino acid sequence of any of SEQ ID NOs: 1 to 11, or the amino acid sequence at positions 338 to 515 of SEQ ID NO: 1, the amino acid sequence at positions 381 to 564 of SEQ ID NO: 2, the amino acid sequence at positions 122 to 309 of SEQ ID NO: 4, the amino acid sequence at positions 1 to 146 of SEQ ID NO: 5, the amino acid sequence at positions 19 to 235 of SEQ ID NO: 6, the amino acid sequence at positions 19 to 205 of SEQ ID NO: 6, the amino acid sequence at positions 10 to 303 of SEQ ID NO: 7, the amino acid sequence at positions 10 to 186 of SEQ ID NO: 7, the amino acid sequence at positions 162 to 426 of SEQ ID NO: 9, or the amino acid sequence at positions 162 to 341 of SEQ ID NO: 9;
(b) a polypeptide including an amino acid sequence provided by substituting, adding, inserting, or deleting one or several amino acids to or from the amino acid sequence of any of SEQ ID NOs: 1 to 11, or the amino acid sequence at positions 338 to 515 of SEQ ID NO: 1, the amino acid sequence at positions 381 to 564 of SEQ ID NO: 2, the amino acid sequence at positions 122 to 309 of SEQ ID NO: 4, the amino acid sequence at positions 1 to 146 of SEQ ID NO: 5, the amino acid sequence at positions 19 to 235 of SEQ ID NO: 6, the amino acid sequence at positions 19 to 205 of SEQ ID NO: 6, the amino acid sequence at positions 10 to 303 of SEQ ID NO: 7, the amino acid sequence at positions 10 to 186 of SEQ ID NO: 7, the amino acid sequence at positions 162 to 426 of SEQ ID NO: 9, or the amino acid sequence at positions 162 to 341 of SEQ ID NO: 9, the polypeptide having catalytic activity for a reaction of deamidating a glutamine residue of a protein; and
(c) a polypeptide including an amino acid sequence having sequence identity of 70% or more with the amino acid sequence of any of SEQ ID NOs: 1 to 11, or the amino acid sequence at positions 338 to 515 of SEQ ID NO: 1, the amino acid sequence at positions 381 to 564 of SEQ ID NO: 2, the amino acid sequence at positions 122 to 309 of SEQ ID NO: 4, the amino acid sequence at positions 1 to 146 of SEQ ID NO: 5, the amino acid sequence at positions 19 to 235 of SEQ ID NO: 6, the amino acid sequence at positions 19 to 205 of SEQ ID NO: 6, the amino acid sequence at positions 10 to 303 of SEQ ID NO: 7, the amino acid sequence at positions 10 to 186 of SEQ ID NO: 7, the amino acid sequence at positions 162 to 426 of SEQ ID NO: 9, or the amino acid sequence at positions 162 to 341 of SEQ ID NO: 9, the polypeptide having catalytic activity for a reaction of deamidating a glutamine residue of a protein.

Item 2. A DNA encoding the protein deamidating enzyme according to item 1.

Item 3. An expression cassette or a recombinant vector comprising the DNA according to item 2.

Item 4. A transformant obtained by transforming a host using the expression cassette or recombinant vector according to item 3.

Item 5. A method for producing a protein deamidating enzyme, including a step of culturing the transformant according to item 4.

Item 6. An enzyme preparation including the protein deamidating enzyme according to item 1.

Item 7. The enzyme preparation according to item 6, which is a modifier for a food or drink or a material for a food or drink, an industrial material, or a pharmaceutical material containing a protein.

Item 8. A method for producing a protein deamidated at a glutamine residue, including a step of allowing the protein deamidating enzyme according to item 1 to act on a protein.

Item 9. The method according to item 8, in which the protein is contained in a food or drink or a material for a food or drink, an industrial material, or a pharmaceutical material.

Item 10. A method for producing a modified food, drink or food or drink material, industrial material, or pharmaceutical material, including a step of allowing the protein deamidating enzyme according to item 1 to act on a food or drink or a material for a food or drink, an industrial material, or a pharmaceutical material containing a protein.

### ADVANTAGES OF THE INVENTION

The present invention provides a novel protein deamidating enzyme.

### EMBODIMENT OF THE INVENTION

As used herein, the term "nonpolar amino acid" includes alanine, valine, leucine, isoleucine, proline, methionine, phenylalanine, and tryptophan. "Non-charged amino acids" include glycine, serine, threonine, cysteine, tyrosine, asparagine, and glutamine. "Acidic amino acids" include aspartic acid and glutamic acid. "Basic amino acids" include lysine, arginine, and histidine.

### 1. Protein Deamidating Enzyme

The protein deamidating enzyme of the present invention is protein deamidating enzymes derived from (1) Halobacteriovorax sp., (2) Halobacteriovoraceae bacterium, (3) Oryza sativa Japonica Group, (4) Acidobacteria bacterium, (5) Deltaproteobacteria bacterium, (6) Rhodospirillaceae bacterium, (7) Flavobacterium alvei, (8) Embleya scabrispora, (9) Lentzea kentuckyensis, (10) Streptomyces chilikensis, and (11) Streptomyces sp., and a protein deamidating enzymes having sequences similar to those of these protein deamidating enzymes.

Specifically, the protein deamidating enzyme of the present invention is constituted of a polypeptide as defined in any one of the following (a) to (c).

(a) A polypeptide including the amino acid sequence of any of SEQ ID NOs: 1 to 11, or the amino acid sequence at positions 338 to 515 of SEQ ID NO: 1, the amino acid sequence at positions 381 to 564 of SEQ ID NO: 2, the amino acid sequence at positions 122 to 309 of SEQ ID NO: 4, the amino acid sequence at positions 1 to 146 of SEQ ID NO: 5, the amino acid sequence at positions 19 to 235 of SEQ ID NO: 6, the amino acid sequence at positions 19 to 205 of SEQ ID NO: 6, the amino acid sequence at positions 10 to 303 of SEQ ID NO: 7, the amino acid sequence at positions 10 to 186 of SEQ ID NO: 7, the amino acid sequence at positions 162 to 426 of SEQ ID NO: 9, or the amino acid sequence at positions 162 to 341 of SEQ ID NO: 9;
(b) a polypeptide including an amino acid sequence provided by substituting, adding, inserting, or deleting one or several amino acids to or from the amino acid sequence of any of SEQ ID NOs: 1 to 11, or the amino acid sequence at positions 338 to 515 of SEQ ID NO: 1, the amino acid sequence at positions 381 to 564 of SEQ ID NO: 2, the amino acid sequence at positions 122 to 309 of SEQ ID NO: 4, the amino acid sequence at positions 1 to 146 of SEQ ID NO: 5, the amino acid sequence at positions 19 to 235 of SEQ ID NO: 6, the amino acid sequence at positions 19 to 205 of SEQ ID NO: 6, the amino acid sequence at positions 10 to 303 of SEQ ID NO: 7, the amino acid sequence at positions 10 to 186 of SEQ ID NO: 7, the amino acid sequence at positions 162 to 426 of SEQ ID NO: 9, or the amino acid sequence at positions 162 to 341 of SEQ ID NO: 9, the polypeptide having catalytic activity for a reaction of deamidating a glutamine residue of a protein; and
(c) a polypeptide including an amino acid sequence having sequence identity of 70% or more with the amino acid sequence of any of SEQ ID NOs: 1 to 11, or the amino acid sequence at positions 338 to 515 of SEQ ID NO: 1, the amino acid sequence at positions 381 to 564 of SEQ ID NO: 2, the amino acid sequence at positions 122 to 309 of SEQ ID NO: 4, the amino acid sequence at positions 1 to 146 of SEQ ID NO: 5, the amino acid sequence at positions 19 to 235 of SEQ ID NO: 6, the amino acid sequence at positions 19 to 205 of SEQ ID NO: 6, the amino acid sequence at positions 10 to 303 of SEQ ID NO: 7, the amino acid sequence at positions 10 to 186 of SEQ ID NO: 7, the amino acid sequence at positions 162 to 426 of SEQ ID NO: 9, or the amino acid sequence at positions 162 to 341 of SEQ ID NO: 9, the polypeptide having catalytic activity for a reaction of deamidating a glutamine residue of a protein.

The protein deamidating enzyme constituted of the polypeptide of (a) to (c) will be described below in detail.

### 1-1. Protein Deamidating Enzyme Constituted of Polypeptide of (a)

### 1-1-1. Protein Deamidating Enzyme Derived from Halobacteriovorax sp.

SEQ ID NO: 1 is a full-length sequence of an example of a protein deamidating enzyme derived from Halobacteriovorax sp. SEQ ID NO: 1 includes a sequence presumed to be a pro-sequence. A protein of another example of the protein deamidating enzyme derived from Halobacteriovorax sp. does not contain an N-terminal sequence including the sequence presumed to be the pro-sequence in SEQ ID NO: 1 and is specifically constituted of the amino acid sequence at positions 338 to 515 of SEQ ID NO: 1.

### 1-1-2. Protein Deamidating Enzyme Derived from Halobacteriovoraceae bacterium

SEQ ID NO: 2 is a full-length sequence of an example of a protein deamidating enzyme derived from Halobacteriovoraceae bacterium. SEQ ID NO: 2 includes a sequence presumed to be a pro-sequence. A protein of another example of the protein deamidating enzyme derived from Halobacteriovoraceae bacterium does not contain an N-terminal sequence including the sequence presumed to be the pro-sequence in SEQ ID NO: 2 and is specifically constituted of the amino acid sequence at positions 381 to 564 of SEQ ID NO: 2.

### 1-1-3. Protein Deamidating Enzyme Derived from Oryza sativa Japonica Group

SEQ ID NO: 3 is a full-length sequence of an example of a protein deamidating enzyme derived from Oryza sativa Japonica Group. SEQ ID NO: 3 can include an N-terminal sequence including a sequence presumed to be a pro-sequence and/or a C-terminal sequence.

### 1-1-4. Protein Deamidating Enzyme Derived from Acidobacteria bacterium

SEQ ID NO: 4 is a full-length sequence of an example of a protein deamidating enzyme derived from Acidobacteria bacterium. SEQ ID NO: 4 includes a sequence presumed to be a pro-sequence. A protein of another example of the protein deamidating enzyme derived from Acidobacteria bacterium does not contain an N-terminal sequence including the sequence presumed to be the pro-sequence in SEQ ID NO: 4 and is specifically constituted of the amino acid sequence at positions 122 to 309 of SEQ ID NO: 4.

### 1-1-5. Protein Deamidating Enzyme Derived from Deltaproteobacteria bacterium

SEQ ID NO: 5 is a full-length sequence of an example of a protein deamidating enzyme derived from Deltaproteobacteria bacterium. SEQ ID NO: 5 includes a C-terminal sequence. A protein of another example of the protein deamidating enzyme derived from Deltaproteobacteria bacterium does not contain the C-terminal sequence in SEQ ID NO: 5 and is specifically constituted of the amino acid sequence, which is the amino acid sequence of SEQ ID NO: 26, at positions 1 to 146 of SEQ ID NO: 5.

### 1-1-6. Protein Deamidating Enzyme Derived from Rhodospirillaceae bacterium

SEQ ID NO: 6 is a full-length sequence of an example of a protein deamidating enzyme derived from Rhodospirillaceae bacterium. SEQ ID NO: 6 includes a sequence presumed to be a pro-sequence and a C-terminal sequence. A protein of another example of the protein deamidating enzyme derived from Rhodospirillaceae bacterium does not contain an N-terminal sequence including the sequence presumed to be the pro-sequence and/or the C-terminal sequence in SEQ ID NO: 6 and is specifically constituted of the amino acid sequence at positions 19 to 235 or the amino acid sequence at positions 19 to 205 of SEQ ID NO: 6.

### 1-1-7. Protein Deamidating Enzyme Derived from Flavobacterium alvei

SEQ ID NO: 7 is a full-length sequence of an example of a protein deamidating enzyme derived from Flavobacterium alvei. SEQ ID NO: 7 includes a sequence presumed to be a pro-sequence and a C-terminal sequence. A protein of another example of the protein deamidating enzyme derived from Flavobacterium alvei does not contain an N-terminal sequence including the sequence presumed to be the pro-sequence and/or the C-terminal sequence in SEQ ID NO: 7 and is specifically constituted of the amino acid sequence at positions 10 to 303 or the amino acid sequence at positions 10 to 186 of SEQ ID NO: 7.

### 1-1-8. Protein Deamidating Enzyme Derived from Embleya scabrispora

SEQ ID NO: 8 is a full-length sequence of an example of a protein deamidating enzyme derived from Embleya scabrispora. SEQ ID NO: 8 can include a pro-sequence and/or a C-terminal sequence.

### 1-1-9. Protein Deamidating Enzyme Derived from Lentzea kentuckyensis

SEQ ID NO: 9 is a full-length sequence of an example of a protein deamidating enzyme derived from Lentzea kentuckyensis. SEQ ID NO: 9 includes a sequence presumed to be a pro-sequence and a C-terminal sequence. A protein of another example of the protein deamidating enzyme derived from Lentzea kentuckyensis does not contain an N-terminal sequence including the sequence presumed to be the pro-sequence and/or the C-terminal sequence in SEQ ID NO: 9 and is specifically constituted of the amino acid sequence at positions 162 to 426 or the amino acid sequence at positions 162 to 341 of SEQ ID NO: 9.

### 1-1-10. Protein Deamidating Enzyme Derived from Streptomyces chilikensis

SEQ ID NO: 10 is a full-length sequence of an example of a protein deamidating enzyme derived from Streptomyces chilikensis. SEQ ID NO: 10 can include a pro-sequence and/or a C-terminal sequence.

### 1-1-11. Protein Deamidating Enzyme Derived from Streptomyces sp.

SEQ ID NO: 11 is a full-length sequence of an example of a protein deamidating enzyme derived from Streptomyces sp. SEQ ID NO: 11 can include a pro-sequence and/or a C-terminal sequence.

### 1-2. Protein Deamidating Enzymes Constituted of Polypeptides of (b) and (c)

The polypeptides of (b) and (c) are protein deamidating enzymes having similar sequences based on the amino acid sequence of the polypeptide of (a).

The modification with the amino acid to be introduced into the polypeptide of (b) may include only one type of modification (such as substitution) from among substitution, addition, insertion, and deletion or may include two or more types of modifications (such as substitution and insertion). In the polypeptide of (b), 1 or more or several amino acids are substituted, added, inserted, or deleted, and the number is, for example, 1 to 10, preferably 1 to 8, 1 to 6, 1 to 5, or 1 to 4, more preferably 1 to 3, particularly preferably 1 or 2 or 1.

The sequence identity of the polypeptide (c) is only required to be 70% or more but is preferably 80% or more, 85% or more, 90% or more, more preferably 95% or more, 97% or more, 98% or more, still more preferably 99% or more, particularly preferably 99.5% or more or 99.8% or more.

Here, in the polypeptide of (c), for example, the sequence identity with the amino acid sequence of SEQ ID NO: 1 is sequence identity calculated as compared with the amino acid sequence of SEQ ID NO: 1. The same applies to the sequence identity with an amino acid sequence represented by the amino acid sequence of any of SEQ ID NOs: 2 to 11, or the amino acid sequence at positions 338 to 515 of SEQ ID NO: 1, the amino acid sequence at positions 381 to 564 of SEQ ID NO: 2, the amino acid sequence at positions 122 to 309 of SEQ ID NO: 4, the amino acid sequence at positions 1 to 146 of SEQ ID NO: 5, the amino acid sequence at positions 19 to 235 of SEQ ID NO: 6, the amino acid sequence at positions 19 to 205 of SEQ ID NO: 6, the amino acid sequence at positions 10 to 303 of SEQ ID NO: 7, the amino acid sequence at positions 10 to 186 of SEQ ID NO: 7, the amino acid sequence at positions 162 to 426 of SEQ ID NO: 9, or the amino acid sequence at positions 162 to 341 of SEQ ID NO: 9. In addition, the "sequence identity" indicates a value of identity of an amino acid sequence obtained using the bl2seq program (Tatiana A. Tatsusova, Thomas L. Madden, FEMS Microbiol. Lett., Vol. 174, p. 247-250, 1999) of BLAST PACKAGE [sgi 32 bit edition, Version 2.0.12; available from National Center for Biotechnology Information (NCBI)]. Parameters may be set to a gap insertion cost value of 11 and a gap extension cost value of 1.

In the polypeptides of (b) and (c), amino acids at positions 376, 415, and 435 in the amino acid sequence of SEQ ID NO: 1; amino acids at positions 421, 464, and 484 in the amino acid sequence of SEQ ID NO: 2; amino acids at positions 43, 91, and 112 in the amino acid sequence of SEQ ID NO: 3; amino acids at positions 165, 215, and 238 in the amino acid sequence of SEQ ID NO: 4; amino acids at positions 28, 77, and 96 in the amino acid sequence of SEQ ID NO: 5; amino acids at positions 60, 105, and 125 in the amino acid sequence of SEQ ID NO: 6; amino acids at positions 41, 97, and 116 in the amino acid sequence of SEQ ID NO: 7; amino acids at positions 251, 295, and 310 in the amino acid sequence of SEQ ID NO: 8; amino acids at positions 203, 247, and 263 in the amino acid sequence of SEQ ID NO: 9; amino acids at positions 238, 282, and 297 in the amino acid sequence of SEQ ID NO: 10; and amino acids at positions 272, 324, and 344 in the amino acid sequence of SEQ ID NO: 11 are considered to contribute to the activity, and it is desirable not to introduce substitutions or deletions at these sites (hereinafter also referred to as "predetermined sites"). That is, in the polypeptide of (b), the site of the amino acid to be substituted, added, inserted, or deleted is preferably a site other than the predetermined sites, and in the polypeptide of (c), the sequence identity is preferably sequence identity in a portion other than the predetermined sites.

In the case where an amino acid substitution is introduced into the polypeptides of (b) and (c), conservative substitution is mentioned as an aspect of the amino acid substitution. That is, in the polypeptides of (b) and (c), examples of the amino acid substitution to be introduced into an amino acid sequence represented by the amino acid sequence of any of SEQ ID NOs: 1 to 11, or the amino acid sequence at positions 338 to 515 of SEQ ID NO: 1, the amino acid sequence at positions 381 to 564 of SEQ ID NO: 2, the amino acid sequence at positions 122 to 309 of SEQ ID NO: 4, the amino acid sequence at positions 1 to 146 of SEQ ID NO: 5, the amino acid sequence at positions 19 to 235 of SEQ ID NO: 6, the amino acid sequence at positions 19 to 205 of SEQ ID NO: 6, the amino acid sequence at positions 10 to 303 of SEQ ID NO: 7, the amino acid sequence at positions 10 to 186 of SEQ ID NO: 7, the amino acid sequence at positions 162 to 426 of SEQ ID NO: 9, or the amino acid sequence at positions 162 to 341 of SEQ ID NO: 9 include substitution with another nonpolar amino acid if the amino acid before substitution is a nonpolar amino acid, substitution with another uncharged amino acid if the amino acid before substitution is an uncharged amino acid, substitution with another acidic amino acid if the amino acid before substitution is an acidic amino acid, and substitution with another basic amino acid if the amino acid before substitution is a basic amino acid.

In the case where an amino acid addition is introduced into the polypeptides of (b) and (c), examples of an aspect of the amino acid addition include addition of a methionine residue to the N-terminal and addition of a tag for purification (such as a binding oligopeptide such as oligohistidine).

The polypeptides of (b) and (c) include not only polypeptides obtained by artificial mutations but also polypeptides generated by naturally occurring mutations (mutants or variants) based on individual differences or species differences of organisms from which the polypeptides are derived.

In the polypeptides of (b) and (c), the "catalytic activity for a reaction of deamidating a glutamine residue of a protein" (hereinafter also referred to as "protein-glutaminase activity") means activity of acting on a protein to degrade an amide group-containing side chain without cleaving peptide bonds or crosslinking the protein, and specifically, the activity can be confirmed by detection of a product associated with deamidation of a glutamine residue. For example, the protein-glutaminase activity can be confirmed by detecting a substrate in which a glutamine residue has been converted to a glutamic acid residue or released ammonia as a product formed by causing a protein deamidating enzyme to act on a peptide of two or more residues (such as N-benzyloxycarbonyl-L-glutaminylglycine (Z-Gln-Gly) or the like) containing a glutamine residue and not containing an asparagine residue as a substrate. In the quantitative determination of the protein-glutaminase activity, the protein-glutaminase activity can be quantitatively calculated on the basis of the amount of the product, preferably the amount of released ammonia. Specifically, the protein-glutaminase activity can be measured by adding 0.1 mL of an enzyme solution having an appropriate concentration to 1 mL of a 0.2-mol/L phosphate buffer (pH 6.5) containing 30 mmol/L of Z-Gln-Gly, carrying out an enzyme reaction at 37°C for 10 minutes, then adding 1 mL of a 0.4-mol/L trichloroacetic acid solution to stop the enzyme reaction, and measuring the ammonia concentration in the supernatant. In the present invention, such enzyme activity as to produce 1 µmol ammonia per minute under this condition is defined as one unit (U) of protein-glutaminase activity.

Specific examples of the polypeptide (b) or (c) include polypeptides having the amino acid sequences of SEQ ID NO: 23 (obtained by adding a methionine residue at the N-terminal of the amino acid sequence at positions 338 to 515 of SEQ ID NO: 1), SEQ ID NO: 24 (obtained by adding a methionine residue at the N-terminal of the amino acid sequence at positions 381 to 564 of SEQ ID NO: 2), SEQ ID NO: 25 (obtained by adding a methionine residue at the N-terminal of the amino acid sequence at positions 122 to 309 of SEQ ID NO: 4), SEQ ID NO: 27 (obtained by adding a methionine residue at the N-terminal of the amino acid sequence at positions 19 to 235 of SEQ ID NO: 6), SEQ ID NO: 28 (obtained by adding a methionine residue at the N-terminal of the amino acid sequence at positions 19 to 205 of SEQ ID NO: 6), SEQ ID NO: 29 (obtained by adding a methionine residue at the N-terminal of the amino acid sequence at positions 10 to 303 of SEQ ID NO: 7), SEQ ID NO: 30 (obtained by adding a methionine residue at the N-terminal of the amino acid sequence at positions 10 to 186 of SEQ ID NO: 7), SEQ ID NO: 31 (obtained by adding a methionine residue at the N-terminal of the amino acid sequence at positions 162 to 426 of SEQ ID NO: 9), and SEQ ID NO: 32 (obtained by adding a methionine residue at the N-terminal of the amino acid sequence at positions 162 to 341 of SEQ ID NO: 9).

The amino acid sequences of SEQ ID NOs: 1 to 11 are referred to as "full-length sequences", while hereinafter the amino acid sequence at positions 338 to 515 of SEQ ID NO: 1, the amino acid sequence at positions 381 to 564 of SEQ ID NO: 2, the amino acid sequence at positions 122 to 309 of SEQ ID NO: 4, the amino acid sequence at positions 1 to 146 of SEQ ID NO: 5, the amino acid sequence at positions 19 to 235 of SEQ ID NO: 6, the amino acid sequence at positions 19 to 205 of SEQ ID NO: 6, the amino acid sequence at positions 10 to 303 of SEQ ID NO: 7, the amino acid sequence at positions 10 to 186 of SEQ ID NO: 7, the amino acid sequence at positions 162 to 426 of SEQ ID NO: 9, and the amino acid sequence at positions 162 to 341 of SEQ ID NO: 9 may be referred to as "non-full-length sequences". In addition, a polypeptide having a full-length sequence may be referred to as a "full-length polypeptide", and a polypeptide having a non-full-length sequence may be referred to as a "non-full-length polypeptide".

### 2. DNA

A DNA (hereinafter may be referred to as a "DNA of the present invention") encoding the protein deamidating enzyme of the present invention can be appropriately prepared and designed by those skilled in the art according to the amino acid sequence of the protein deamidating enzyme of the present invention.

The base sequence of the DNA encoding the protein deamidating enzyme of the present invention can be appropriately designed by those skilled in the art according to the amino acid sequence described regarding to the protein deamidating enzyme of the present invention.

Specifically, examples of the DNA encoding the protein deamidating enzyme of the present invention include a DNA represented by any of the following (i) to (iii).

(i) A DNA including the base sequence of any of SEQ ID NOs: 12 to 22, or the base sequence at positions 1012 to 1548 of SEQ ID NO: 12, the base sequence at positions 1141 to 1695 of SEQ ID NO: 13, the base sequence at positions 364 to 930 of SEQ ID NO: 15, the base sequence at positions 1 to 438 of SEQ ID NO: 16, the base sequence at positions 55 to 708 of SEQ ID NO: 17, the base sequence at positions 55 to 615 of SEQ ID NO: 17, the base sequence at positions 28 to 912 of SEQ ID NO: 18, the base sequence at positions 28 to 558 of SEQ ID NO: 18, the base sequence at positions 484 to 1281 of SEQ ID NO: 20, or the base sequence at positions 484 to 1023 of SEQ ID NO: 20;
(ii) A DNA including a base sequence of a DNA that hybridizes under stringent conditions to a DNA having a base sequence complementary to the base sequence of any of SEQ ID NOs: 12 to 22, or the base sequence at positions 1012 to 1548 of SEQ ID NO: 12, the base sequence at positions 1141 to 1695 of SEQ ID NO: 13, the base sequence at positions 364 to 930 of SEQ ID NO: 15, the base sequence at positions 1 to 438 of SEQ ID NO: 16, the base sequence at positions 55 to 708 of SEQ ID NO: 17, the base sequence at positions 55 to 615 of SEQ ID NO: 17, the base sequence at positions 28 to 912 of SEQ ID NO: 18, the base sequence at positions 28 to 558 of SEQ ID NO: 18, the base sequence at positions 484 to 1281 of SEQ ID NO: 20, or the base sequence at positions 484 to 1023 of SEQ ID NO: 20; and
(iii) A DNA including a base sequence having homology of 70% or more with the base sequence of any of SEQ ID NOs: 12 to 22, or the base sequence at positions 1012 to 1548 of SEQ ID NO: 12, the base sequence at positions 1141 to 1695 of SEQ ID NO: 13, the base sequence at positions 364 to 930 of SEQ ID NO: 15, the base sequence at positions 1 to 438 of SEQ ID NO: 16, the base sequence at positions 55 to 708 of SEQ ID NO: 17, the base sequence at positions 55 to 615 of SEQ ID NO: 17, the base sequence at positions 28 to 912 of SEQ ID NO: 18, the base sequence at positions 28 to 558 of SEQ ID NO: 18, the base sequence at positions 484 to 1281 of SEQ ID NO: 20, or the base sequence at positions 484 to 1023 of SEQ ID NO: 20.

The DNAs of (i) to (iii) will be described below in detail.

### 2-1. DNA of (i)

### 2-1-1. DNA Encoding Protein Deamidating Enzyme Derived from Halobacteriovorax sp.

Examples of the base sequence encoding the amino acid sequence (the full-length sequence of an example of the protein deamidating enzyme derived from Halobacteriovorax sp.) of SEQ ID NO: 1 include the base sequence of SEQ ID NO: 12; and examples of the base sequence encoding the amino acid sequence at positions 338 to 515 (a sequence without N-terminal sequence of a protein of another example of the protein deamidating enzyme derived from Halobacteriovorax sp.) of the amino acid sequence of SEQ ID NO: 1 include the base sequence at positions 1012 to 1548 of SEQ ID NO: 12.

### 2-1-2. DNA Encoding Protein Deamidating Enzyme Derived from Halobacteriovoraceae bacterium

Examples of the base sequence encoding the amino acid sequence (the full-length sequence of an example of the protein deamidating enzyme derived from Halobacteriovoraceae bacterium) of SEQ ID NO: 2 include the base sequence of SEQ ID NO: 13; and examples of the base sequence encoding the amino acid sequence at positions 381 to 564 (a sequence without N-terminal sequence of a protein of another example of the protein deamidating enzyme derived from Halobacteriovoraceae bacterium) of SEQ ID NO: 2 include the base sequence at positions 1141 to 1695 of SEQ ID NO: 13.

### 2-1-3. DNA Encoding Protein Deamidating Enzyme Derived from Oryza sativa Japonica Group

Examples of the base sequence encoding the amino acid sequence (the full-length sequence of an example of the protein deamidating enzyme derived from Oryza sativa Japonica Group) of SEQ ID NO: 3 include the base sequence of SEQ ID NO: 14.

### 2-1-4. DNA Encoding Protein Deamidating Enzyme Derived from Acidobacteria bacterium

Examples of the base sequence encoding the amino acid sequence (the full-length sequence of an example of the protein deamidating enzyme derived from Acidobacteria bacterium) of SEQ ID NO: 4 include the base sequence of SEQ ID NO: 15; and examples of the base sequence encoding the amino acid sequence at positions 122 to 309 (a sequence without N-terminal sequence of a protein of another example of the protein deamidating enzyme derived from Acidobacteria bacterium) of SEQ ID NO: 4 include the base sequence at positions 364 to 930 of SEQ ID NO: 15.

### 2-1-5. DNA Encoding Protein Deamidating Enzyme Derived from Deltaproteobacteria bacterium

Examples of the base sequence encoding the amino acid sequence (the full-length sequence of an example of the protein deamidating enzyme derived from Deltaproteobacteria bacterium) of SEQ ID NO: 5 include the base sequence of SEQ ID NO: 16; and examples of the base sequence encoding the amino acid sequence at positions 1 to 146 (a sequence without C-terminal sequence of a protein of another example of the protein deamidating enzyme derived from Deltaproteobacteria bacterium) of SEQ ID NO: 5 include the base sequence at positions 1 to 438 of SEQ ID NO: 16.

### 2-1-6. DNA Encoding Protein Deamidating Enzyme Derived from Rhodospirillaceae bacterium

Examples of the base sequence encoding the amino acid sequence (the full-length sequence of an example of the protein deamidating enzyme derived from Rhodospirillaceae bacterium) of SEQ ID NO: 6 include the base sequence of SEQ ID NO: 17; and examples of the base sequence encoding the amino acid sequence at positions 19 to 235 (a sequence without N-terminal sequence of a protein of another example of the protein deamidating enzyme derived from Rhodospirillaceae bacterium) of SEQ ID NO: 6 or the amino acid sequence at positions 19 to 205 (a sequence without N-terminal sequence and C-terminal sequence of a protein of another example of the protein deamidating enzyme derived from Rhodospirillaceae bacterium) of SEQ ID NO: 6 respectively include the base sequence at positions 55 to 708 or the base sequence at positions 55 to 615 of SEQ ID NO: 17.

### 2-1-7. DNA Encoding Protein Deamidating Enzyme Derived from Flavobacterium alvei

Examples of the base sequence encoding the amino acid sequence (the full-length sequence of an example of the protein deamidating enzyme derived from Flavobacterium alvei) of SEQ ID NO: 7 include the base sequence of SEQ ID NO: 18; and examples of the base sequence encoding the amino acid sequence at positions 10 to 303 (a sequence without N-terminal sequence of a protein of another example of the protein deamidating enzyme derived from Flavobacterium alvei) of SEQ ID NO: 7 or the amino acid sequence at positions 10 to 186 (a sequence without N-terminal sequence and C-terminal sequence of a protein of another example of the protein deamidating enzyme derived from Flavobacterium alvei) of SEQ ID NO: 7 respectively include the base sequence at positions 28 to 912 or the base sequence at positions 28 to 558 of SEQ ID NO: 18.

### 2-1-8. DNA Encoding Protein Deamidating Enzyme Derived from Embleya scabrispora

Examples of the base sequence encoding the amino acid sequence (the full-length sequence of an example of the protein deamidating enzyme derived from Embleya scabrispora) of SEQ ID NO: 8 include the base sequence of SEQ ID NO: 19.

### 2-1-9. DNA Encoding Protein Deamidating Enzyme Derived from Lentzea kentuckyensis

Examples of the base sequence encoding the amino acid sequence (the full-length sequence of an example of the protein deamidating enzyme derived from Lentzea kentuckyensis) of SEQ ID NO: 9 include the base sequence of SEQ ID NO: 20; and examples of the base sequence encoding the amino acid sequence at positions 162 to 426 (a sequence without N-terminal sequence of a protein of another example of the protein deamidating enzyme derived from Lentzea kentuckyensis) of SEQ ID NO: 9 or the amino acid sequence at positions 162 to 341 (a sequence without N-terminal sequence and C-terminal sequence of a protein of another example of the protein deamidating enzyme derived from Lentzea kentuckyensis) of SEQ ID NO: 9 respectively include the base sequence at positions 484 to 1281 or the base sequence at positions 484 to 1023 of SEQ ID NO: 20.

### 2-1-10. DNA Encoding Protein Deamidating Enzyme Derived from Streptomyces chilikensis

Examples of the base sequence encoding the amino acid sequence (the full-length sequence of an example of the protein deamidating enzyme derived from Streptomyces chilikensis) of SEQ ID NO: 10 include the base sequence of SEQ ID NO: 21.

### 2-1-11. DNA Encoding Protein Deamidating Enzyme Derived from Streptomyces sp.

Examples of the base sequence encoding the amino acid sequence (the full-length sequence of an example of the protein deamidating enzyme derived from Streptomyces sp.) of SEQ ID NO: 11 include the base sequence of SEQ ID NO: 22.

### 2-2. DNAs of (ii) and (iii)

The DNAs of (ii) and (iii) are DNAs having similar sequences based on the base sequence of the DNA of (i).

For the DNA of (ii) above, the "stringent conditions" refer to conditions of incubation for 4 hours to overnight at 50°C to 65°C in 6 × SSC (1 × SSC contains 0.15 M of NaCl and 0.015 M of sodium citrate, and is pH 7.0) containing 0.5% of SDS, 5 ×Denhardt's solution [Denhartz's, 0.1% of bovine serum albumin (BSA), 0.1% of polyvinylpyrrolidone, and 0.1% of Ficoll 400], and 100 µg/ml of salmon sperm DNA.

Hybridization under the stringent conditions is specifically performed by the following method. That is, a nylon membrane on which a DNA library or a cDNA library is immobilized is prepared, and the nylon membrane is blocked at 65°C in a prehybridization solution containing 6 × SSC, 0.5% of SDS, 5 × Denhardt's solution, and 100 µg/ml of salmon sperm DNA. Each probe labeled with ³²P is then added, and the membrane is incubated overnight at 65°C. This nylon membrane is washed in 6 × SSC at room temperature for 10 minutes, in 2 × SSC containing 0.1% of SDS at room temperature for 10 minutes, and in 0.2 × SSC containing 0.1% of SDS at 45°C for 30 minutes, and autoradiography is then performed, so that the DNA that has specifically hybridized to the probe can be detected.

The homology of the DNA of (iii) is only required to be 70% or more but is preferably 80% or more, 90% or more, more preferably 95% or more, 97% or more, 98% or more, particularly preferably 99% or more.

Here, the "homology" of the base sequence indicates a value of identity obtained using the bl2seq program (Tatiana A. Tatsusova, Thomas L. Madden, FEMS Microbiol. Lett., Vol. 174, 247-250, 1999) of BLAST PACKAGE [sgi 32 bit edition, Version 2.0.12; available from The National Center for Biotechnology Information (NCBI)]. Parameters may be set to a gap insertion cost value of 11 and a gap extension cost value of 1.

In the examples of the DNAs of (ii) and (iii), at least one of base sequences encoding a start codon, a stop codon, and a tag for purification (such as a binding oligopeptide such as oligohistidine) may be further added to the DNA of (i).

More specific examples of the DNAs of (ii) and (iii) include DNAs having the base sequences of SEQ ID NO: 33 (a base sequence in which a base sequence encoding a methionine residue is further added to the 5' end of the base sequence at positions 1012 to 1548 of SEQ ID NO: 12), SEQ ID NO: 34 (a base sequence in which a base sequence encoding a methionine residue is further added to the 5' end of the base sequence at positions 1141 to 1695 of SEQ ID NO: 13), SEQ ID NO: 35 (a base sequence in which a base sequence encoding a methionine residue is further added to the 5' end of the base sequence at positions 364 to 930 of SEQ ID NO: 15), SEQ ID NO: 36 (a base sequence in which a stop codon is further added to the 3' end of the base sequence at positions 1 to 438 of SEQ ID NO: 16), SEQ ID NO: 37 (a base sequence in which a base sequence encoding a methionine residue is further added to the 5' end of the base sequence at positions 55 to 708 of SEQ ID NO: 17), SEQ ID NO: 38 (a base sequence in which a base sequence encoding a methionine residue is further added to the 5' end and a stop codon is further added to the 3' end of the base sequence at positions 55 to 615 of SEQ ID NO: 17), SEQ ID NO: 39 (a base sequence in which a base sequence encoding a methionine residue is further added to the 5' end of the base sequence at positions 28 to 912 of SEQ ID NO: 18), SEQ ID NO: 40 (a base sequence in which a base sequence encoding a methionine residue is further added to the 5' end and a stop codon is further added to the 3' end of the base sequence at positions 28 to 558 of SEQ ID NO: 18), SEQ ID NO: 41 (a base sequence in which a base sequence encoding a methionine residue is further added to the 5' end of the base sequence at positions 484 to 1281 of SEQ ID NO: 20), and SEQ ID NO: 42 (a base sequence in which a base sequence encoding a methionine residue is further added to the 5' end and a stop codon is further added to the 3' end of the base sequence at positions 484 to 1023 of SEQ ID NO: 20).

### 2-3. Preparation of DNA

The DNA of the present invention can be obtained, for example, by obtaining a region encoding at least any of the polypeptides of (a) to (c) by PCR or the like using the DNA encoding any of the polypeptides of (a) to (c) as a template. The DNA encoding the protein deamidating enzyme of the present invention can also be artificially synthesized by a gene synthesis method.

In addition, in the case where a specific mutation is introduced into a specific site of the base sequence, a mutation introduction method is known, and for example, a site-directed mutagenesis method for DNA or the like can be used. As a specific method for converting a base in DNA, for example, a commercially available kit can be used.

The base sequence of a DNA into which a mutation has been introduced can be examined using a DNA sequencer. Once the base sequence is determined, the DNA encoding the protein deamidating enzyme can be obtained by chemical synthesis, PCR using a cloned probe as a template, or hybridization using a DNA fragment having the base sequence as a probe.

In addition, a mutant form of DNA encoding the protein deamidating enzyme and having a function equivalent to that before mutation can be synthesized by a site-directed mutagenesis method or the like. In addition, in order to introduce a mutation into the DNA encoding the protein deamidating enzyme, a known method such as the Kunkel method, the Gapped duplex method, and the megaprimer PCR method can be used.

The DNA of the present invention is preferably one in which the codon usage frequency is optimized for the host. For example, in the case where a colon bacillus is used as the host, a DNA in which the codon usage frequency is optimized for the colon bacillus is suitable.

### 3. Expression Cassette or Recombinant Vector

The expression cassette or recombinant vector (hereinafter also referred to as the "expression cassette of the present invention" or the "recombinant vector of the present invention") containing the DNA encoding the protein deamidating enzyme of the present invention contains the DNA encoding the protein deamidating enzyme of the present invention. The expression cassette or recombinant vector of the present invention can be obtained by linking a promoter and a terminator to the DNA of the present invention or by inserting the expression cassette of the present invention or the DNA of the present invention into an expression vector.

The expression cassette or recombinant vector of the present invention may further contain, as a controllable factor, a transcription element such as an enhancer, the CCAAT box, the TATA box, and an SPI site as necessary, in addition to a promoter and a terminator. These controllable factors may be operably linked to the DNA of the present invention. Operably linking means that various controllable factors that regulate the DNA of the present invention and the DNA of the present invention are linked in a state where they can operate in a host cell.

In the case where a non-full-length polypeptide is to be expressed, the expression cassette of the present invention or the recombinant vector of the present invention may be designed to directly express the non-full-length polypeptide or, as described below, may be designed so that the terminal sequence can be cleaved with a protease after the full-length polypeptide containing a protease recognition sequence is once expressed.

In the case where the expression cassette of the present invention or the recombinant vector of the present invention is designed so that the terminal sequence can be cleaved with a protease after a full-length polypeptide containing a protease recognition sequence is once expressed, the expression cassette or recombinant vector of the present invention can be configured to contain a base sequence encoding the protease recognition sequence and a base sequence encoding an N-terminal sequence and/or a C-terminal sequence in combination. Specifically, the expression cassette or recombinant vector of the present invention can be configured to contain a base sequence encoding a recognition sequence for a specific protease between the base sequence encoding the N-terminal sequence including a pre-sequence and/or a pro-sequence and the base sequence encoding the non-full-length polypeptide and the like and/or between the base sequence encoding the non-full-length polypeptide and the like and the base sequence encoding the C-terminal sequence, in addition to the base sequence encoding the non-full-length polypeptide and the like. In this case, after the full-length polypeptide is expressed, by specifically removing the N-terminal sequence and/or the C-terminal sequence using the specific protease, a protein deamidating enzyme constituted of the non-full-length polypeptide can be obtained.

As the expression vector, those constructed for genetic recombination from a phage, a plasmid, or a virus capable of autonomously proliferate in the host are suitable. Such expression vectors are known, and those skilled in the art can appropriately select and use an appropriate combination with a host cell. For example, in the case of using a microorganism as the host, examples include pBluescript (pBS) II SK (-) (manufactured by Stratagene), pSTV vectors (manufactured by Takara Bio Inc.), pUC vectors (manufactured by Takara Bio Inc.), pET vectors (manufactured by Merck KGaA), pGEX vectors (manufactured by GE HealthCare), pCold vectors (manufactured by Takara Bio Inc.), pHY300PLK (manufactured by Takara Bio Inc.), pUB110 (Mckenzie, T. et al., 1986, Plasmid 15 (2), pp. 93-103), pBR322 (manufactured by Takara Bio Inc.), pRS403 (manufactured by Stratagene), and pMW218/219 (manufactured by Nippon Gene Co., Ltd.). In the case where an algae or a microalgae is used as the host, examples include pUC19 (manufactured by Takara Bio Inc.), P-66 (Chlamydomonas Center), P-322 (Chlamydomonas Center), pPha-T1 (see Yangmin Gong, et al., Journal of Basic Microbiology, 2011, vol. 51, pp. 666-672), and pJET1 (manufactured by Cosmo Bio Co., Ltd.). In the case of using a plant cell as the host, examples include pRI vectors (manufactured by Takara Bio Inc.), pBI vectors (manufactured by Clontech Laboratories, Inc.), and IN3 vectors (manufactured by Inplanta Innovations, Inc.).

### 4, Transformant

A transformant (hereinafter may be referred to as a "transformant of the present invention") is obtained by transforming a host using the expression cassette or recombinant vector of the present invention.

The host used for the production of the transformant is not particularly limited as long as it can be subjected to introduction of a gene, can autonomously proliferate, and can express the trait of the gene of the present invention. Suitable examples thereof include bacteria belonging to the genus Escherichia such as colon bacilli (Escherichia cofi), the genus Bacillus such as Bacillus subtilis, and the genus Pseudomonas such as Pseudomonas putida; actinomycetes; yeasts; and microorganisms such as filamentous fungi, and in addition, animal cells, insect cells, plants, and the like may also be used. Among them, colon bacilli are particularly preferable.

The host used for the production of the transformant may be [1] Halobacteriovorax sp., [2] Halobacteriovoraceae bacterium, [3] cells of Oryza sativa Japonica Group, [4] Acidobacteria bacterium, [5] Deltaproteobacteria bacterium, [6] Rhodospirillaceae bacterium, [7] Flavobacterium alvei, [8] Embleya scabrispora, [9] Lentzea kentuckyensis, [10] Streptomyces chilikensis, or [11] Streptomyces sp., which are bacteria or cells from which the protein deamidating enzyme of the present invention is derived.

The transformant of the present invention can be obtained by introducing the expression cassette or recombinant vector of the present invention into the host. The place where the DNA of the present invention is introduced is not particularly limited as long as a target gene can be expressed and may be on a plasmid or on a genome. Specific examples of the method for introducing the expression cassette of the present invention or the recombinant vector of the present invention include the recombinant vector method and the genome editing method.

Conditions for introducing the expression cassette or recombinant vector of the present invention into the host may be appropriately set according to the type of the host and the like. In the case where the host is a microorganism, examples include a method using a competent cell by calcium ion treatment, the electroporation method, the spheroplast method, and the lithium acetate method. In the case where the host is an animal cell, examples include the electroporation method, the calcium phosphate method, and the lipofection method. In the case where the host is an insect cell, examples include the calcium phosphate method, the lipofection method, and the electroporation method. In the case where the host is a plant cell, examples include the electroporation method, the Agrobacterium method, the particle gun method, and the PEG method.

### 5. Method for Producing Protein Deamidating Enzyme

The present invention also provides a method for producing the above protein deamidating enzyme. The protein deamidating enzyme can be produced by culturing the transformant of the present invention. In addition, the protein deamidating enzyme can also be produced by culturing the bacteria or cells of [1] to [11] themselves (untransformed), from which the protein deamidating enzyme is derived.

The culture conditions of the transformant of the present invention or the bacteria or cells from which the enzyme is derived are appropriately set in consideration of the nutritional physiological properties of the host or the bacteria or cells from which the enzyme is derived, and liquid culture is preferable. In the case of industrial production, aeration agitation culture is preferable.

The transformant of the present invention or the bacteria or cells from which the enzyme is derived are cultured, and the culture supernatant or the cultured bacterial cells or cultured cells are recovered by a method such as centrifugation of the culture solution. In the case where the protein deamidating enzyme of the present invention is accumulated in cultured bacterial cells or cultured cells, the bacterial cells or cells are treated by a mechanical method using ultrasonic waves, a French press, or the like or with a lytic enzyme such as lysozyme and, if necessary, solubilized by using an enzyme such as protease or a surfactant such as sodium dodecyl sulfate (SDS), whereby a water-soluble fraction containing the protein deamidating enzyme of the present invention can be obtained.

In addition, an appropriate expression vector and host may be selected to allow the expressed protein deamidating enzyme of the present invention to be secreted into the culture solution.

In one embodiment of the method for producing a protein deamidating enzyme, a full-length protein can be directly expressed by selecting an expression cassette or recombinant vector configured to be capable of expressing a full-length polypeptide as the expression cassette or recombinant vector used for producing the transformant, and in another embodiment, a non-full-length protein can be directly expressed by selecting an expression cassette or recombinant vector configured to be capable of expressing a non-full-length polypeptide as the expression cassette or recombinant vector used for producing the transformant.

Further, examples of the embodiment include the case of selecting an expression cassette or recombinant vector configured to contain a base sequence encoding the full-length polypeptide and the case of selecting an expression cassette or recombinant vector configured to contain a base sequence encoding the non-full-length polypeptide in combination with a base sequence encoding a protease recognition sequence and a base sequence encoding an N-terminal sequence and/or a C-terminal sequence as the expression cassette or recombinant vector used for producing the transformant. The method for producing the protein deamidating enzyme of the present invention in these cases may further include a step of removing the N-terminal sequence and/or the C-terminal sequence (step of obtaining a non-full-length polypeptide) for the purpose of obtaining the non-full-length polypeptide from the full-length polypeptide once expressed as described below.

In one example of the step of obtaining a non-full-length polypeptide, as the expression cassette or recombinant vector used for producing the transformant, one configured to contain the base sequence encoding the full-length polypeptide can be selected, and removal of the N-terminal sequence can be performed, for example, by treating a protein deamidating enzyme with a processing enzyme. Examples of the processing enzymes include proteases. Specific examples of the protease include serine proteases such as subtilisin, chymotrypsin, and trypsin; cysteine protease such as papain, bromelain, caspase, and calpain; acidic proteases such as pepsin and cathepsin; and metalloproteases such as thermolysin.

As another example of the step of obtaining a non-full-length polypeptide, as the expression cassette or recombinant vector used for producing the transformant, one configured to contain the base sequence encoding the non-full-length polypeptide in combination with a base sequence encoding a protease recognition sequence and a base sequence encoding an N-terminal sequence and/or a C-terminal sequence is selected, a full-length polypeptide containing a recognition sequence of a specific protease is expressed between the base sequence encoding the N-terminal sequence and the base sequence encoding the non-full-length polypeptide and/or between the base sequence encoding the non-full-length polypeptide and the base sequence encoding the C-terminal sequence, and then the N-terminal sequence and/or the C-terminal sequence can be specifically removed using the specific protease.

The culture solution, the water-soluble fraction, or the protease-treated product containing the protein deamidating enzyme of the present invention obtained as described above may be subjected to the purification treatment as it is or may be subjected to the purification treatment after the protein deamidating enzyme of the present invention in the culture solution, the water-soluble fraction, or the protease-treated product is concentrated.

The concentration can be performed by, for example, vacuum concentration, membrane concentration, salting-out treatment, fractional precipitation with a hydrophilic organic solvent (such as methanol, ethanol, and acetone), or the like.

The purification treatment of the protein deamidating enzyme of the present invention can be performed, for example, by appropriately combining methods such as gel filtration, hydrophobic chromatography, ion exchange chromatography, and affinity chromatography.

The protein deamidating enzyme of the present invention thus purified may be pulverized by lyophilization, vacuum drying, spray drying, or the like as necessary.

### 6. Enzyme Preparation

The protein deamidating enzyme of the present invention can be provided in the form of an enzyme agent. Therefore, the present invention also provides an enzyme agent containing the protein deamidating enzyme of the present invention.

The content of the protein deamidating enzyme of the present invention in the enzyme agent of the present invention is not particularly limited and can be appropriately set within a range in which protein-glutaminase activity is exhibited.

In addition to the protein deamidating enzyme of the present invention, the enzyme agent of the present invention may contain other components to the extent that the effect of the present invention is not affected. Examples of the other components include enzymes other than the protein deamidating enzyme of the present invention, additives, and residues of the culture generated during the production method.

The other enzymes can be appropriately determined depending on the use thereof, and examples thereof include amylases (α-amylase, β-amylase, and glucoamylase), glucosidases (α-glucosidase and β-glucosidase), galactosidases (α-galactosidase and β-galactosidase), proteases (acidic proteases, neutral proteases, and alkaline protease), peptidases (leucine peptidase and aminopeptidase), lipases, esterases, cellulases, phosphatases (acid phosphatases and alkaline phosphatases), nucleases, deaminases, oxidases, dehydrogenases (other than the above-described active ingredients), glutaminases, pectinases, catalases, dextranases, transglutaminases, protein deamidating enzymes (other than the above-described protein deamidating enzyme of the present invention), and pullulanases. These other enzymes may be contained alone or in combination of two or more thereof.

The additives can be appropriately determined according to the use of the polypeptide of the present invention and the formulation form of the enzyme agent, and examples thereof include excipients, buffers, suspending agents, stabilizers, preservatives, antiseptics, and physiological saline. Examples of the excipients include starch, dextrin, maltose, trehalose, lactose, D-glucose, sorbitol, D-mannitol, sucrose, and glycerol. Examples of the buffers include phosphates, citrates, and acetates. Examples of the stabilizers include propylene glycol and ascorbic acid. Examples of the preservatives include phenol, benzalkonium chloride, benzyl alcohol, chlorobutanol, and methylparaben. Examples of the antiseptics include ethanol, benzalkonium chloride, p-oxybenzoic acid, and chlorobutanol. These other additives may be contained alone or in combination of two or more thereof.

Examples of the residues of the culture include components derived from the culture medium, contaminant proteins, bacterial cell components, and cell components.

The preparation form of the enzyme agent of the present invention is not particularly limited, and examples thereof include liquid and solid forms (powder, granules, etc.). The enzyme agents in these preparation forms can be prepared by a generally known method.

Specific examples of the enzyme agent of the present invention include modifiers for foods and drinks containing proteins or raw materials of the foods and drinks. Other specific examples of the enzyme agent of the present invention include reagents for protein analysis and/or research, such as a reagent for quantifying the amide content of a protein and a reagent for solubilizing a protein.

### 7. Utilization of Protein Deamidating Enzyme

The protein deamidating enzyme of the present invention can be used for any application utilizing the action of deamidation of a glutamine residue in a protein. Accordingly, the present invention also provides a method for producing a protein deamidated at a glutamine residue, including a step of allowing the protein deamidating enzyme of the present invention to act on a protein.

The protein (substrate protein) deamidated by the protein deamidating enzyme is not particularly limited as long as it is a protein containing a glutamine residue. The length of the substrate protein is not particularly limited as long as it includes two (dipeptide) or more residues, and the substrate protein includes not only a protein but also a peptide. The substrate protein includes both natural and artificial proteins. In the case where the substrate protein is a natural protein, the source of the substrate protein may be either an animal or a plant. Furthermore, the substrate protein may be a protein contained in a food or drink or a raw material thereof or may be a protein used as an analysis target or a research target.

Since the protein deamidating enzyme can generate a negatively charged carboxyl group by deamidation of a glutamine residue in the protein, the protein deamidating enzyme can change (modify) the characteristics of the protein by an action similar to the known action of a known protein deamidating enzyme, such as increasing the solubility and water dispersibility of the protein and improving the emulsifying power, emulsion stability, foaming property, and foam stability of the protein. Therefore, the protein deamidating enzyme is particularly useful in the case where a protein contained in a food or drink, or a material for a food or drink (raw material of a food or drink) is used as a substrate. Furthermore, the protein deamidating enzyme is useful not only for a food or drink, or a material for a food or drink but in the case where a protein contained in an industrial material used in the fiber field, the cosmetic field, or the like and a protein contained in a pharmaceutical material are used as substrates. Accordingly, the present invention also provides a method for producing a modified food or drink or material for food or drink, industrial material, or pharmaceutical material, including a step of allowing the protein deamidating enzyme of the present invention to act on a food or drink or a material for a food or drink, an industrial material, or a pharmaceutical material containing a protein.

In the method for producing a protein deamidated at a glutamine residue and the method for producing a modified food or drink or material for food or drink, industrial material, or pharmaceutical material of the present invention, the conditions (the amount of enzyme, the reaction time, the reaction temperature, the reaction pH, etc.) for the reaction between the protein deamidating enzyme of the present invention and the substrate protein are not particularly limited as long as deamidation is achieved to a desired degree, and those skilled in the art can appropriately set the reaction conditions according to various conditions such as the type and/or purity of the protein deamidating enzyme of the present invention, the type and/or concentration of the substrate protein, and/or the desired degree of deamidation.

### EXAMPLES

The present invention will be specifically described below with reference to examples, but the present invention is not to be construed as being limited to the following examples.

Protein deamidating enzymes (Examples 1-1, 1-2, 2-1, 2-2, 3, 4-1, 4-2, 5-1, 5-2, 6-1, 6-2, 6-3, 7-1, 7-2, 7-3, 8, 9-1, 9-2, 9-3, 10, and 11) having the amino acid sequences shown in Table 1 were prepared. Table 1 also shows the amino acid sequences of the prepared protein deamidating enzymes and the base sequences of DNAs encoding the respective amino acid sequences.

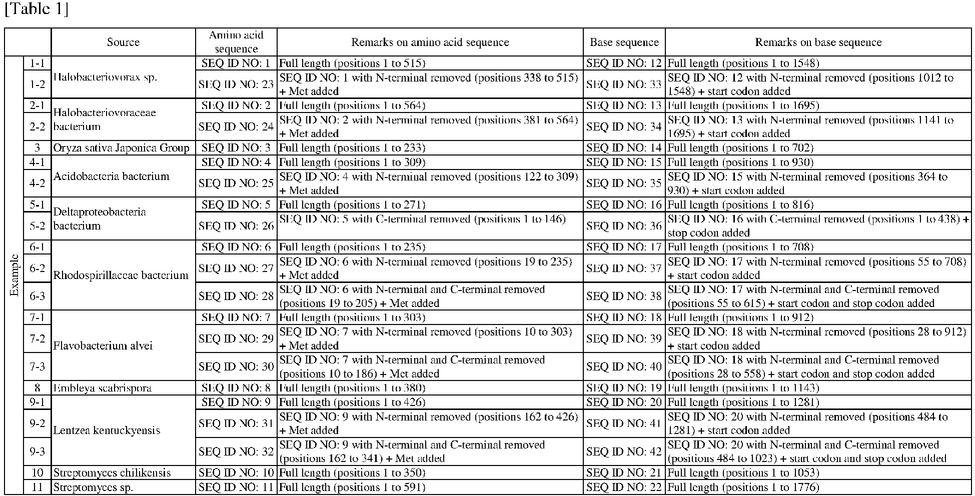

Each protein deamidating enzyme was prepared through the following procedure. The base sequences of SEQ ID NOs: 12 to 22 and 33 to 42 were each cloned into pUC19, pET21a, or pColdI to produce a plasmid. The obtained plasmid was introduced into BL21 (DE3) to obtain a transformant. The transformant was inoculated into the TB + Amp medium or the Over Night Expression TB (OETB) + Amp medium and shaken and cultured overnight at 37°C. When the TB + Amp medium was used, 1.0 mM of IPTG was added at the timing when the OD660 was 0.4. The bacterial cells in the obtained culture solution were collected, and then the bacterial cells were disrupted to collect a soluble fraction (protein deamidating enzyme fraction).

Among the obtained soluble fractions (protein deamidating enzyme fractions), those of examples shown in Table 2-1 to Table 2-11 were evaluated for deamidation activity through the following procedure. After 10 µL of the soluble fraction was mixed with a substrate solution (5 mM of Z-Gln-Gly, 0.0023% of Triton X-100, and 0.2 M of monopotassium disodium phosphate, pH 6.5), the reaction was allowed to proceed overnight at 37°C. After adding 10 µL of hippuric acid (6.25 mM of hippuric acid and 0.2 M of monopotassium disodium phosphate, pH 6.5) as an internal standard to the reaction solution and mixing the solutions, the sample filtered by MF was subjected to HPLC analysis under the following conditions to quantify the peak intensities of Z-Gln-Gly (substrate) and Z-Glu-Gly (product), and the degree of deamidation activity was evaluated using the ratio. The results are shown in Table 2-1 to Table 2-11.

### (HPLC Conditions)

Column: ZORBAX SB-C18 2.1 × 50 mm, 1.8 µm
Solvent A: 0.1% TFA/ H2O Solvent B : 0.1% TFA/ acetonitrile
Program: 0-0.3 min_B : 10%
0.3-2.0 min_B: 10-25%
2.0-2.2 min_B: 25%
2.2-3.0 min_B: 100%
Injection volume: 2 µL
Flow rate: 1.0 mL/min
Detection: UV 205 nm

**[Table 2-1]**

| (1) Halobacteriovoraceae bacterium | | |
|---|---|---|
| | | Proportion (%) |
| Comparative Example 1 | Without enzyme | 0.0 |
| Example 1-1 | Full length | 7.5 |
| Example 1-2 | N-terminal sequence removed | 40.2 |

**[Table 2-2]**

| (2) Halobacteriovorax sp. | | |
|---|---|---|
| | | Proportion (%) |
| Comparative Example 2 | Without enzyme | 0.0 |
| Example 2-2 | N-terminal sequence removed | 3.3 |

**[Table 2-3]**

| (3) Oryza sativa Japonica Group | | |
|---|---|---|
| | | Proportion (%) |
| Comparative Example 3 | Without enzyme | 0.0 |
| Example 3 | Full length | 3.9 |

**[Table 2-4]**

| (4) Acidobacteria bacterium | | |
|---|---|---|
| | | Proportion (%) |
| Comparative Example 4 | Without enzyme | 0.0 |
| Example 4-1 | Full length | 0.7 |
| Example 4-2 | N-terminal sequence removed | 0.4 |

**[Table 2-5]**

| (5) Deltaproteobacteria bacterium | | |
|---|---|---|
| | | Proportion (%) |
| Comparative Example 5 | Without enzyme | 0.0 |
| Example 5-2 | C-terminal sequence removed | 0.4 |

**[Table 2-6]**

| (6) Rhodospirillaceae bacterium | | |
|---|---|---|
| | | Proportion (%) |
| Comparative Example 6 | Without enzyme | 0.0 |
| Example 6-1 | Full length | 0.4 |
| Example 6-2 | N-terminal sequence removed | 0.4 |
| Example 6-3 | N-terminal sequence and C-terminal sequence removed | 0.2 |

**[Table 2-7]**

| (7) Flavobacterium alvei | | |
|---|---|---|
| | | Proportion (%) |
| Comparative Example 7 | Without enzyme (control) | 0.0 |
| Example 7-1 | Full length | 0.6 |
| Example 7-2 | N-terminal sequence removed | 0.1 |
| Example 7-3 | N-terminal sequence and C-terminal sequence removed | 0.03 |

**[Table 2-8]**

| (8) Embleya scabrispora | | | |
|---|---|---|---|
| | | | Proportion (%) |
| | Comparative Example 8 | Without enzyme (control) | 0.0 |
| | Example 8 | Full length | 0.3 |

**[Table 2-9]**

| (9) Lentzea kentuckyensis | | | |
|---|---|---|---|
| | | | Proportion (%) |
| | Comparative Example 9 | Without enzyme | 0.0 |
| | Example 9-2 | N-terminal sequence removed | 0.1 |
| | Example 9-3 | N-terminal sequence and C-terminal sequence removed | 2.5 |

**[Table 2-10]**

| (10) Streptomyces chilikensis | | | |
|---|---|---|---|
| | | | Proportion (%) |
| | Comparative Example 10 | Without enzyme (control) | 0.0 |
| | Example 10 | Full length | 0.4 |

**[Table 2-11]**

| (11) Streptomyces sp. | | | |
|---|---|---|---|
| | | | Proportion (%) |
| | Comparative Example 11 | Without enzyme (control) | 0.0 |
| | Example 11 | Full length | 0.5 |

It was confirmed that all of the enzymes shown in Tables 2-1 to 2-11 produced peaks of the product deamidated at a glutamine residue. That is, it was recognized that all of the enzymes shown in Tables 2-1 to 2-11 were protein deamidating enzymes having protein-glutaminase activity. In particular, it was also confirmed that the protein-glutaminase activity of the protein deamidating enzyme of Example 1-2 was remarkably high, and the degree thereof was about 2.7 times as high as that of a commercially available protein deamidating enzyme (derived from Chryseobacterium proteolyticum).

## Claims

1. A protein deamidating enzyme comprising a polypeptide as defined in any of (a) to (c) below:
(a) a polypeptide including an amino acid sequence of any of SEQ ID NOs: 1 to 11, or an amino acid sequence at positions 338 to 515 of SEQ ID NO: 1, an amino acid sequence at positions 381 to 564 of SEQ ID NO: 2, an amino acid sequence at positions 122 to 309 of SEQ ID NO: 4, an amino acid sequence at positions 1 to 146 of SEQ ID NO: 5, an amino acid sequence at positions 19 to 235 of SEQ ID NO: 6, an amino acid sequence at positions 19 to 205 of SEQ ID NO: 6, an amino acid sequence at positions 10 to 303 of SEQ ID NO: 7, an amino acid sequence at positions 10 to 186 of SEQ ID NO: 7, an amino acid sequence at positions 162 to 426 of SEQ ID NO: 9, or an amino acid sequence at positions 162 to 341 of SEQ ID NO: 9;
(b) a polypeptide including an amino acid sequence provided by substituting, adding, inserting, or deleting one or several amino acids to or from the amino acid sequence of any of SEQ ID NOs: 1 to 11, or the amino acid sequence at positions 338 to 515 of SEQ ID NO: 1, the amino acid sequence at positions 381 to 564 of SEQ ID NO: 2, the amino acid sequence at positions 122 to 309 of SEQ ID NO: 4, the amino acid sequence at positions 1 to 146 of SEQ ID NO: 5, the amino acid sequence at positions 19 to 235 of SEQ ID NO: 6, the amino acid sequence at positions 19 to 205 of SEQ ID NO: 6, the amino acid sequence at positions 10 to 303 of SEQ ID NO: 7, the amino acid sequence at positions 10 to 186 of SEQ ID NO: 7, the amino acid sequence at positions 162 to 426 of SEQ ID NO: 9, or the amino acid sequence at positions 162 to 341 of SEQ ID NO: 9, the polypeptide having catalytic activity for a reaction of deamidating a glutamine residue of a protein; and
(c) a polypeptide including an amino acid sequence having sequence identity of 70% or more with the amino acid sequence of any of SEQ ID NOs: 1 to 11, or the amino acid sequence at positions 338 to 515 of SEQ ID NO: 1, the amino acid sequence at positions 381 to 564 of SEQ ID NO: 2, the amino acid sequence at positions 122 to 309 of SEQ ID NO: 4, the amino acid sequence at positions 1 to 146 of SEQ ID NO: 5, the amino acid sequence at positions 19 to 235 of SEQ ID NO: 6, the amino acid sequence at positions 19 to 205 of SEQ ID NO: 6, the amino acid sequence at positions 10 to 303 of SEQ ID NO: 7, the amino acid sequence at positions 10 to 186 of SEQ ID NO: 7, the amino acid sequence at positions 162 to 426 of SEQ ID NO: 9, or the amino acid sequence at positions 162 to 341 of SEQ ID NO: 9, the polypeptide having catalytic activity for a reaction of deamidating a glutamine residue of a protein.

2. A DNA encoding the protein deamidating enzyme according to claim 1.

3. An expression cassette or a recombinant vector comprising the DNA according to claim 2.

4. A transformant obtained by transforming a host using the expression cassette or recombinant vector according to claim 3.

5. A method for producing a protein deamidating enzyme, comprising a step of culturing the transformant according to claim 4.

6. An enzyme preparation comprising the protein deamidating enzyme according to claim 1.

7. The enzyme preparation according to claim 6, which is a modifier for a food or drink or a material for a food or drink, an industrial material, or a pharmaceutical material containing a protein.

8. A method for producing a protein deamidated at a glutamine residue, comprising a step of allowing the protein deamidating enzyme according to claim 1 to act on a protein.

9. The method according to claim 8, wherein the protein is contained in a food or drink or a material for a food or drink, an industrial material, or a pharmaceutical material.

10. A method for producing a modified food, drink or food or drink material, industrial material, or pharmaceutical material, comprising a step of allowing the protein deamidating enzyme according to claim 1 to act on a food or drink or a material for a food or drink, an industrial material, or a pharmaceutical material containing a protein.
